# EUROPEAN PATENT SPECIFICATION

(11) **EP 1 512 746 B1**
(45) Date of publication and mention of the grant of the patent: **07.10.2009**
(21) Application number: 03018478.2
(22) Date of filing: 14.08.2003
(51) Int. Cl.: C12N 15/67, C12N 15/63, C12N 15/85, C07K 1/00

(54) **Method for the production of a polypeptide, RNA or other compound in tumor tissue**
Methode zur Herstellung eines Polypeptides, einer RNA oder einer anderen Verbindung in Tumorgewebe
Méthode pour la production d'un polypeptide, d'un ARN ou d'un autre composé dans des tissus tumoraux

(43) Date of publication of application: 09.03.2005
(73) Proprietor: Genelux Corporation, San Diego, California 92109 (US)
(72) Inventor: Szalay, Aladar A., Highland, California 92346 (US)
(74) Representative: Baldock, Sharon Claire

(56) References cited:
- US-B1- 6 589 531
- ZHENG LI-MOU ET AL: "Tumor amplified protein expression therapy: Salmonella as a tumor-selective protein delivery vector" ONCOLOGY RESEARCH, vol. 12, no. 3, 2000, pages 127-135, XP009020983 ISSN: 0965-0407

## Description

The present invention relates to a method for the production of a polypeptide, RNA or other compound comprising (a) injecting a microorganism or cell containing a cloned DNA sequence encoding the desired polypeptide or RNA into an animal bearing a tumour and (b) isolating the desired polypeptide, RNA or compound from the tumour tissue. The method of the invention is also useful for the concomitant production of a desired antigen and an antibody directed to said antigen, wherein said method comprises the additional step of isolating antibodies from the serum of the animal bearing the tumour that produces the antigen.

Until now, the engineering and large scale prosecution of recombinant proteins, e.g., in tissue cultures, has been time consuming and expensive, prohibiting in many cases the widespread use of these proteins, e.g., in medicine. Recent developments in protein engineering and production technologies have contributed to overcome at least some of the problems. For example, using molecular farming, i.e. the production of recombinant preteins in transgenic plants can be used to synthesize foreigh proteins, e.g., antibodies and vaccines on an agricultural scale. However, this approach also has some drawbacks, e.g., in the case of recombinantly produced human proteins the glycosylation pattern of the protein differs from the pattern f the natural protein.

Attempts have been made to overcome this problem.

US 6,589,531 B1 discloses recombinant yellow fever viruses which comprise exogenous nucleotide sequences encoding exogenous amino acid sequences. Infection of a host cell with the recombinant yellow fever virus provides for expression of the exogenous nucleic acid and production of an antigenic polypeptide.

Zheng et al (Oncol. Res. [2000] Vol 12 p 127-135) discloses the use of salmonella as a tumour-selective protein delivery vector. This was demonstrated using green fluorescent protein or cytosine deaminase, which after administration to tumour bearing mice, could be detected in tumours but not in liver.

Therefore, it is the object of the present invention to provide a means for the efficient recombinant production of biologically active proteins which overcomes disadvantages of the approaches for recombinant production of proteins presently used.

The solution to the above technical problem has been achieved by providing the embodiments characterized in the claims. In EP-A1 1 281 772 it is diclosed that when vaccinia virus (LIVP strain) carrying the light emitting fusion gene construct rVV-ruc-gfp was injected intravenously into nude mice, the virus particles were found to be cleared from all internal organs within 4 days, as determined by extinction of light emission. In contrast, when the fate of the injected vaccinia virus was similarly followed in nude mice bearing tumors grown from subcutaneously implanted C6 rat glioma cells, virus particles were found to be retained over time in the tumor tissues, resulting in lasting light emission. The presence and amplification of the virus-encoded fusion proteins in the same tumor were monitored in live animals by observing GFP fluorescence under a stereomicroscope and by collecting luciferase-catalyzed light emission under a low-light video-imaging camera. Tumor-specific light emission was detected 4 days after viral injection in nude mice carrying subcutaneous C6 glioma implants. Tumor accumulation of rVV-ruc-gfp virus particles was also seen in nude mice carrying subcutaneous tumors developed from implanted PC-3 human prostate cells, and in mice with orthotopically implanted MCF-7 human breast tumors. Further, intracranial C6 rat glioma cell implants in immunocompetent rats and MB-49 human bladder tumor cell implants in C57 mice were also targeted by the vaccinia virus. In addition to primary breast tumors, small metastatic tumors were also detected externally in the contralateral breast region, as well as in nodules on the exposed lung surface, suggesting metastasis to the contralateral breast and lung. In summary, it was shown that light-emitting cells or microorganims, e.g. vaccinia virus can be used to detect and treat primary and metastatic tumors.

Similar results were obtained with light-emitting bacteria *(Salmonella, Vibrio, Listeria, E. coli)* which were injected intravenously into mice and which could be visualized in whole animals under a low light imager immediately. No light emission was detected twenty-four hours after bacterial injection in both athymic (nu/nu) mice and immunocompetent C57 mice as a result of clearing by the immune system. In nude mice baring tumors developed from implanted C6 glioma cells, light emission was abolished from the animal entirely twenty-four hours after delivery of bacteria, similar to mice without tumors. However, forty-eight hours post-injection, unexpectedly, a strong, rapidly increasing light emission originated only from the tumor regions was observed. This observation indicated a continuous bacterial replication in the tumor tissue. The extent of light emission was dependent on the bacterial strain used. The homing-in process together with the sustained light emission was also demonstrated in nude mice carrying prostate, bladder, and breast tumors. In addition to primary tumors, metastatic tumors could also be visualized as exemplified in the breast tumor model. Tumor-specific light emission was also observed in immunocompetent C57 mice with bladder tumors as well as in Lewis rats with brain glioma implants. Once in the tumor, the light-emitting bacteria were not observed to be released into the circulation and to re-colonize subsequently implanted tumors in the same animal. Further, mammalian cells expressing the Ruc-GFP fusion protein, upon injection into the bloodstream, were also found to home into and propagate in glioma tumors. These findings opened the way for designing multifunctional viral vectors useful for the detection of tumors based on signals like light emission, for suppression of tumor development and angiogenesis signaled by, e.g., light extinction and the development of bacterium- and mammalian cell-based tumor targeting systems in combination with therapeutic gene constructs for the treatment of cancer. These systems have the following advantages: (a) They target the tumor specifically without affecting normal tissue; (b) the expression and secretion of the therapeutic gene constructs are, preferably, under the control of an inducible promoter, enabling secretion to be switched on or off; and (c) the location of the delivery system inside the tumor can be verified by direct visualization before activating gene expression and protein delivery.

During the experiments leading to the present invention it has been found that the system described above being based on the accmulation of bacteria, viruses etc. in tumors can be used for the simple, quick, and inexpensive production of proteins and other biological compounds originating from cloned DNA sequences. It has also been found that this system is useful for the concomitant overproduction of polypeptides, RNA or other biological compounds (in tumor tissue) and antibodies against those compounds (in the serum) of the same animal. It was shown that after intravenous injection, vaccinia virus enters the tumor of an animal and, due to its immunoprivileged state, replicates preferentially in the tumor tissues and thereby overproduces of the inserted gene encoded protein in the tumors. After harvesting the tumor tissues, the localized and overexpressed protein could be isolated by a simple procedure from tumor homogenates. In addition, based on the findings that only 0.2 to 0.3% of the desired proteins produced in the tumor were found in the blood stream of the same animal, a simultaneous vaccination of the mouse and efficient antibody production against the overproduced protein was achieved. Thus, serum from the same mouse (or any other animal) can be harvested and used as mouse-derived antibodies against the proteins or other products overproduced in the tumor.

The advantages of the system of the present invention are:
(a) No production of a transgenic animal carrying the novel polypeptide ancoding cassette is required;
(b) the tumor system is more efficient than tissue culture;
(c) proteins interfering with animal development and other toxic proteins may be overproduced in tumors without negative effects to the host animal;
(d) the combined system is fast: Within 4 to 6 weeks from cDNA cloning to protein and antisera purification;
(e) the system is relatively inexpensive and can be scaled up easily;
(f) correct protein folding and modifications can be achieved;
(g) high antigenicity may be achieved which is beneficial for better antibody production; and
(h) species-specific-cell-based production of proteins, e.g., in mice, with tumors as fermentors is achieved.

The system of the present invention can be illustrated by the following scheme:

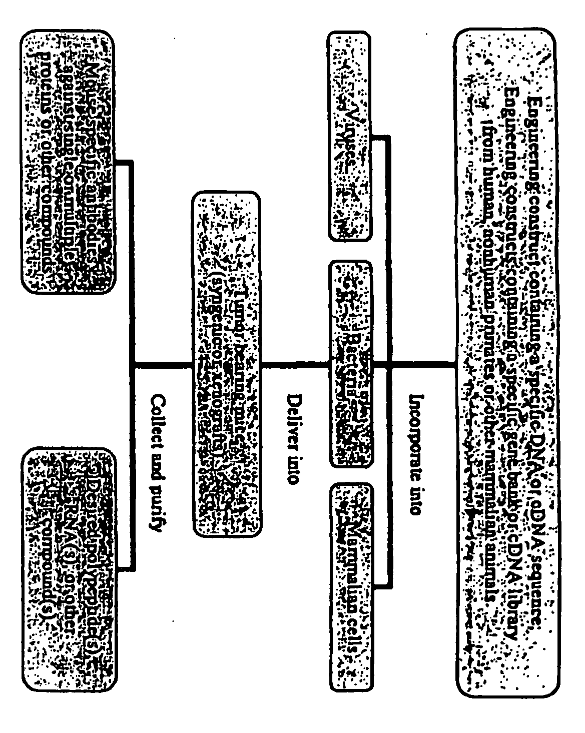

Accordingly, in one embodiment, the present invention relates to a method of producing a desired polypeptide, RNA or compound, said method comprising the following steps: (a) injecting a microorganism or cell containing a cloned DNA sequence encoding the desired polypeptide or RNA into an animal bearing a tumor; (b) harvesting the tumor tissue from said animal; and (c) isolating the desired polypeptide, RNA or compound from said tumor tissue.

Th principal steps of the method of the present invention can be summarized as follows (shown for a particual embodiment, i.e. vaccinia virus additionally containing a gene encoding a light-emitting protein):
(1) Insertion of the desired DNA or cDNA into the vaccinia virus genome;
(2) modification of the vaccinia virus genome with light-emitting protein construct as expression marker;
(3) recombination and virus assembly in cell culture;
(4) screening of individual viral particles carrying inserts followed by large scale virus production and concentration;
(5) injection of the viral particles into mice or other animals bearing tumors of human, non-human primate or other mammalian origins;
(6) verification of viral replication and protein overproduction in animals based on light emission;
(7) harvest of tumor tissues and, optionally, the blood (separately); and
(8) purification of overexpressed proteins from tumors and, optionally, antisera from blood using conventional methods.

As used herein, the term "compound" refers to any compound which is produced in the tumor due to the presence of the recombinant polypeptide, e.g., a compound which is generated by the recombinant polypeptide (e.g., enzyme) and the cellular machinery of the tumor.

Any microorganism or cell is useful for the method of the present invention, provided that they replicate in the animal, are not pathogenic for the animal e.g. attenuated and, are recognized by the immune system of the animal, etc. Suitable micoroganisms and cells are, e.g., disclosed in EP-A1 1 281 772 and EP-A1 1 281 767.

The person skilled in the art also knows how to generate animals carrying the desired tumor (see, e.g., EP-A1 1 281 767 or EP-A1 1 281 777). The tumor tissue is surgically removed from the animal. After homogenisation of the tumor tissue, the desired polypeptide, RNA or other biological compound can be purified according to established methods. For example, in the case of a recombinant polypeptide, the polypeptide might contain a his-tag and can be purified via column chromatography. The time necessary for accumulation of sufficient amounts of the polypetide etc. in the tumor of the animal depends on many factors, e.g., the kind of animal, kind of tumor etc. and can be determined by the skilled person by routine experimentation. In general, the desired polypeptide expression can be detected two days after virus injection. The expression peaks approximately two weeks after injection, and lasts up to two months.

In another embodiment, the present invention relates to a method of producing a desired antibody, said method comprising the following steps: (a) injecting a microorganism or cell containing a DNA sequence encoding an antigen into an animal bearing a tumor; and (b) isolating the antibody directed to said antigen from the serum obtained from said animal. The antibodies directed to said antigen can be isolated and purified according to well known methods. Antibodies that are directed against specific contaminating antigens (e.g. bacteria antigens) can be removed by adsorption, and the antibodies directed against the target antigen can be separated from contaminating antibodies by affinity purification, e.g., by immuno affinity chromatography using the recombinant antigen as the ligand of the column.

In a further emobidment, the present invention relates to a method of simultaneously producing a desired polypeptide, RNA or compound and an antibody directed to said polypeptide, RNA or compound, said method comprising the following steps: (a) injecting a microorganism or cell containing a cloned DNA sequence encoding the desired polypeptide or RNA into an animal bearing a tumor; (b) harvesting the tumor tissue from said animal; (c) isolating the desired polypeptide, RNA or compound from said tumor tissue; and (d) isolating the antibody directed to said polypeptide, RNA or compound from the serum obtained from said animal. This approach is particularly useful for generating polypeptides and/or antibodies against the polypeptides which are toxic or unstable, or which require species specific cellular environment for correct folding or modifications.

In a preferred embodiment of the method of the present invention, said microorganism or cell furthermore contains a DNA sequence encoding a detectable protein, preferably a luminescent or fluorescent protein, or a protein capable of inducing a detectable signal. As used herein, the term "DNA sequence encoding a luminescent and/or fluorescent protein" also comprises a DNA sequence encoding a luminescent and fluorescent protein as fusion protein.

Preferably, for transfecting the microorgaisms or cells the DNA sequences encoding the desired polypeptide etc. and, optionally, a DNA sequence encoding a detectable protein, preferably a luminescent or fluorescent protein, or a protein capable of inducing a detectable signal, the DNA sequences are present in a vector or an expression vector. A person skilled in the art is familiar with examples thereof. The DNA sequences can also be contained in a recombinant virus containing appropriate expression cassettes. Suitable viruses that may be used in the method of the present invention include baculovirus, vaccinia, sindbis virus, Sendai virus, adenovirus, an AAV virus or a parvovirus, such as MVM or H-1. The vector may also be a retrovirus, such as MoMULV, MoMuLV, HaMuSV, MuMTV, RSV or GaLV. For expression in mammalian cells, a suitable promoter is e.g. human cytomegalovirus "immediate early promoter" (pCMV). Furthermore, tissue and/or organ specific promoters are useful. Preferably, the DNA sequences are operatively linked with a promoter allowing high expression. Such promoters, e.g. inducible promoters, are well-known to the person skilled in the art.

For generating the above described DNA sequences and for constructing expression vectors or viruses which contain said DNA sequences, it is possible to use general methods known in the art. These methods include e.g. in vitro recombination techniques, synthetic methods and in vivo recombination methods as described in Sambrook et al., Molecular Cloning, A Laboratory Manual, 2nd edition (1989) Cold Spring Harbor Laboratory Press, Cold Spring Harbor, NY, for example. Methods of transfecting cells, of phenotypically selecting transfectants and of expressing the DNA sequences by using the above described vectors are known in the art.

The person skilled in the art knows DNA sequences encoding luminescent or fluorescent proteins that can be used in the method of the present invention. During the past decade, the identification and isolation of structural genes encoding light-emitting proteins from bacterial luciferase from Vibrio *harveyi* (Belas et al., Science 218 (1982), 791-793) and from *Vibrio fischerii* (Foran and Brown, Nucleic acids Res. 16 (1988), 177), firefly luciferase (de Wet et al., Mol. Cell. Biol. 7 (1987), 725-737), aequorin from *Aequorea victoria* (Prasher et al., Biochem. 26 (1987), 1326-1332), *Renilla* luciferase from *Renilla reniformis* (Lorenz et al., PNAS USA 88 (1991), 4438-4442) and green fluorescent protein from *Aequorea victoria* (Prasher et al., Gene 111 (1987), 229-233) have been described that allow the tracing of bacteria or viruses based on light emission. Transformation and expression of these genes in bacteria allows detection of bacterial colonies with the aid of the low light imaging camera or individual bacteria under the fluorescent microscope (Engebrecht et al., Science 227 (1985), 1345-1347; Legocki et al., PNAS 83 (1986), 9080-9084; Chalfie et al., Science 263 (1994), 802-805).

Luciferase genes have been expressed in a variety of organisms. Promoter activation based on light emission, using *lux AB* fused to the nitrogenase promoter, was demonstrated in Rhizobia residing within the cytoplasm of cells of infected root nodules by low light imaging (Legocki et al., PNAS 83 (1986), 9080-9084; O'Kane et al., J. Plant Mol. Biol. 10 (1988), 387-399). Fusion of the *lux A* and *lux B* genes resulted in a fully functional luciferase protein (Escher et al., PNAS 86 (1989), 6528-6532). This fusion gene *(Fab2)* was introduced into *Bacillus subtilis* and *Bacillus megatherium* under the xylose promoter and then fed into insect larvae and was injected into the hemolymph of worms. Imaging of light emission was conducted using a low light video camera. The movement and localization of pathogenic bacteria in transgenic arabidopsis plants, which carry the pathogen-activated PAL promoter-bacterial luciferase fusion gene construct, was demonstrated by localizing *Pseudomonas* or *Ervinia spp.* infection under the low light imager as well as in tomato plant and stacks of potatoes (Giacomin and Szalay, Plant Sci. 116 (1996), 59-72).

All of the luciferases expressed in bacteria require exogenously added substrates such as decanal or coelenterazine for light emission. In contrast, while visualization of GFP fluorescence does not require a substrate, an excitation light source is needed. More recently, the gene cluster encoding the bacterial luciferase and the proteins for providing decanal within the cell, which includes *luxCDABE* was isolated from *Xenorhabdus luminescens* (Meighen and Szittner, J. Bacteriol. 174 (1992), 5371-5381) and *Photobacterium leiognathi* (Lee et al., Eur. J. Biochem. 201 (1991), 161-167) and transferred into bacteria resulting in continuous light emission independent of exogenously added substrate (Fernandez-Pinas and Wolk, Gene 150 (1994), 169-174). Bacteria containing the complete *lux* operon sequence, when injected intraperitoneally, intramuscularly, or intravenously, allowed the visualization and localization of bacteria in live mice indicating that the luciferase light emission can penetrate the tissues and can be detected externally (Contag et al., Mol. Microbiol. 18 (1995), 593-603).

Thus, in a further preferred embodiment of the method of the present invention the luminescent or fluorescent protein is a luciferase, green fluorescent protein (GFP) or red fluorescent protein (RFP).

In a particularly preferred embodiment, the microorganism or cell of the method of the present invention additionally contains a gene encoding a substrate for the luciferase. In an even more preferred embodiment, the microorganism or cell of the method of the present invention contains a *ruc-gfp* expression cassette which contains the *Renilla* luciferase (ruc) and Aequorea gfp cDNA sequences under the control of a strong synthetic early/late (PE/L) promoter of *Vaccinia* or contains the *luxCDABE cassette.*

Preferably, the microorganism is a bacterium, e.g. attenuated bacterium. Particularly preferred is attenuated *Salmonella thyphimurium*, attenuated *Vibrio cholerae or* attenuated *Listeria monocytogenes* or *E.coli.* Alternatively, viruses such as vaccinia virus, AAV, a retrovirus etc. are also useful for the methods of the present invention. Preferably, the virus is vaccinia virus.

Preferably, the cell of the method of the present invention is a mammalian cell.

Any laboratory animal can be used for the method of the present invention, e.g., mice, rats, rabbits, guinea pigs, pigs, sheep and horses, with mice being preferred.

Preferably, the tumor of the laboratory animal is generated by implanting tumor cells into said animal. Generally speaking, for the production of a desired polypeptide, RNA, or compound, any solid tumor type can be used, with a fast growing tumor type being preferred, e.g. C6 rat glioma or HCT116 human colon carcinoma. For the production of a desired antibody, a relatively slow growing tumor type is preferred, e.g. HT1080 human fibrosarcoma and GI-101A human breast carcinoma. For T-independent antibody production, nu-/nu-mice bearing allogenic tumor or xenografts can be used; while for T-dependent antibody production, immuno-competent mice with syngenic tumors may be the choice.

### Brief description of the drawing:

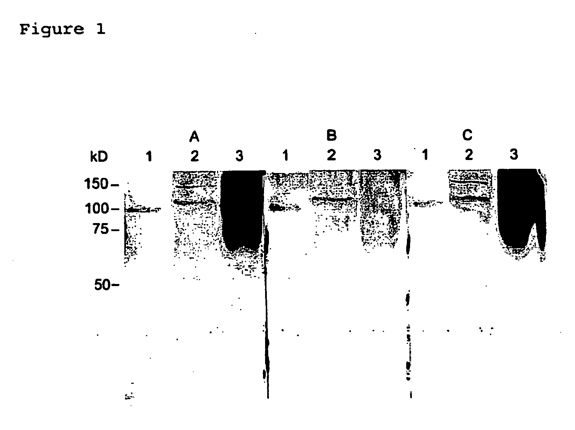

Figure 1 shows the production of Production of Anti β-Galactosidase via Vaccinia Virus Delivered *lac*Z in Tumor Bearing Mice: β-galactosidase from Roche (lane 1), cell lysate from RVGL7 infected CV-1 cells (lane 2), and the tumor lysate from mouse #143, 14 days after infected with RVGL7 (lane 3) were loaded in triplicates and separated in a 10% SDS-polyacrylamide gel. The immuno-blotting was performed with 1:3000 either mouse monoclonal anti β-galactosidase (panel A), or mouse serum taken from either mouse # 116 (panel B) or #119 (panel C). The antigen detection was carried out using Goat Anti Mouse IgG-HRP, 1:3000, and the amplified Opti-4CN Detection Kit.

The invention is explained by the following example:

### Example 1: Production of β-Galactosidase and Anti β-Galactosidase via Vaccinia Virus Delivered lacz in Tumor Bearing Mice

Thirty five athymic nu/nu mice (5 weeks old, 25g, male) were included in the experiment to study biodistribution and tumor targeting of vaccinia virus (strain LIVP) with different deletions in the genome. Mice were divided into 7 groups with 5 in each group as presented in Table 1.

**Table 1. Experiment Design**

| **Group** | **N of mice** | **Tumor implanted** | **Virus injected** | **Locus-genes inserted** |
|---|---|---|---|---|
| 1 | 5 | None | VGL | wt LIVP |
| 2 | 5 | C6, s.c. 5x10⁵ cells | VGL | wt LIVP |
| 3 | 5 | C6, s.c. 5x10⁵ cells | RVGL1 | N-*luc*, *lacZ* |
| 4 | 5 | C6, s.c. 5x10⁵ cells | RVGL5 | *HA-lacZ* |
| 5 | 5 | C6, s.c. 5x10⁵ cells | RVGL7 | TK-*egfp, lacZ* |
| 6 | 5 | C6, s.c. 5x10⁵ cells | RVGL8 | NotI-*lacZ* |
| 7 | 5 | C6, s.c. 5x10⁵ cells | RVGL19 | TK-*rTrf, lacZ*; NotI-RG |

C6 glioma was subcutaneously developed in Group 2 to 7. Five days after tumor cell implantation (5x10⁵ cells /mouse), each animal was treated with 0.1 ml of viruses at the multiplicity of infection (MOI) 1x10⁷ via tail vein injection. Two weeks after virus injection, all mice were sacrificed and blood samples were collected. Various organs and tumors were also taken from animals for virus titration and β-galactosidase analysis. The β-galactosidase analysis was carried out using Galacto-Light Plus system (Applied Biosystems), a chemiluminescent reporter gene assay system for the detection of β-galactosidase, according to manufactures instructions.

In non-tumorous mice as well as in tumorous mice injected with wild type vaccinia virus (without reporter genes and without β-galactosidase gene) no β-galactosidase expression could be detected in organs, blood and tumor samples. By contrast, in the tumors of mice infected with β-galactosidase expressing virus, high amounts of β-galactosidase were obtained. β-galactosidase was also detected in blood samples as shown in Table 2, even though there was no virus recovered from the same samples.

**Table 2. Production of β-galactosidase by vaccinia virus in tumor and blood from tumor bearing mice (day 14 after virus injection)**

| **Group** | **Virus injected** | **β-gal in tumor (µg/mg of total protein)** | **β-gal in serum (µg/mg of total protein)** | **Estimated total β-gal/ tumor (µg)** | **Estimated total β- gal in 5ml blood (µg)** |
|---|---|---|---|---|---|
| 3 | RVGL1 | 1.59 ± 0.41 | 1.38x10⁻² ± 1.09x10⁻² | 489.84 | 4.00 |
| 4 | RVGL5 | 1.51 ± 0.37 | 1.16x10⁻² ± 1.08x10⁻² | 330.21 | 3.62 |
| 5 | RVGL7 | 1.35 ± 0.59 | 0.95x10⁻² ± 1.47x10⁻² | 616.60 | 1.83 |
| 6 | RVGL8 | 1.81 ± 0.42 | 0.86x10⁻² ± 0.33x10⁻² | 962.36 | 2.38 |
| 7 | RVGL19 | 1.30 ± 0.44 | 0.26x10⁻² ± 0.16x10⁻² | 463.75 | 0.60 |

To determine if the amount of β-galactosidase presented in mouse blood was sufficient to elicit antibody production, sera taken from two mice (mouse #116 from Group 5, and #119 from Group 6) were selected as the source for primary antibodies against β-galactosidase in Western analysis. β-galactosidase from *E. coli* (Roche, 567 779) was used as the antigen standard, and the mouse monoclonal anti β-galactosidase from *E. coli* (Sigma, G6282) was used as the antibody positive control. The total protein obtained from CV-1 cells 24 hours after infection with RVGL7 at MOI of 1 pfu/cell was used in the Western analysis, along with the tumor protein sample taken from mouse #143, which was also treated with RVGL7.

The protein samples were prepared in triplicates, each set including the β-galactosidase antigen standard, the cell lysate from RVGL7 infected CV-1 cells, and the tumor lysate from mouse #143. All protein samples were separated in a 10% polyacrylamide gel, and transferred to NitroBind nitrocellulose membrane (MSI) using BioRad semidry blotting system. Immuno-blotting was performed with either 1:3000 mouse monoclonal anti β-galactosidase, or 1:3000 mouse serum taken from either mouse #116 or #119, and 1:3000 Goat AntiMouse IgG-HRP (BioRad). Amplified Opti-4CN Detection Kit (BioRad) was used for antigen detection.

As shown in Figure 1, mouse sera taken from mouse #116 and #119 give similar results as compared to commercial mouse anti β-galactosidase, indicating both tumor bearing mice produced antibodies against β-galactosidase.

## Claims

1. A method of producing a desired polypeptide, RNA molecule or compound, said method comprising the following steps:
(a) injecting a microorganism or cell containing a cloned DNA sequence, which encodes the desired polypeptide or RNA molecule or coding for production of the compound, into a non-human animal bearing a tumor, wherein:
the tumor is a solid tumor; and
the microorganism or cell accumulates in the tumor; and
(b) after sacrificing the animal, harvesting the tumor tissue from said animal; and
(c) isolating the desired polypeptide, RNA molecule or compound from said tumor tissue.

2. A method of producing a desired antibody, said method comprising the following steps:
(a) injecting a microorganism or cell containing a cloned DNA sequence encoding an antigen into a non-human animal bearing a tumor, wherein:
the microorganism or cell accumulates in tumors and expresses the antigen in the tumors; and
the tumor is a solid tumor; and
(b) after sacrificing the animal, isolating the antibody directed to said antigen from the serum obtained from said animal.

3. A method of simultaneously producing a desired polypeptide, RNA molecule or compound and an antibody directed to said polypeptide, RNA molecule or compound, said method comprising the following steps:
(a) injecting a microorganism or cell containing a cloned DNA sequence, which encodes the desired polypeptide, RNA molecule or codes for production of the compound , into a non-human animal bearing a tumor, wherein:
the microorganism or cell accumulates in tumors; and
the tumor is a solid tumor; and
(b) after sacrificing the animal, harvesting, the tumor tissue from said animal;
(c) isolating the desired polypeptide, RNA molecule or compound from said tumor tissue; and
(d) isolating the antibody directed to said polypeptide, RNA molecule or compound from the serum obtained from said animal.

4. The method of any one of claims 1 to 3, wherein said microorganism or cell furthermore contains a DNA molecule encoding a detectable protein or a protein capable of inducing a detectable signal.

5. The method of claim 4, wherein said protein capable of inducing a detectable signal is a luminescent and/or fluorescent protein.

6. The method of claim 5, wherein said luminescent or fluorescent protein is luciferase, RFP or GFP.

7. The method of claim 6, wherein said microorganism or cell additionally contains a gene encoding a substrate for a luciferase.

8. The method of any one of claims 1 to 7, wherein said microorganism is a bacterium or a virus.

9. The method of claim 8, wherein said virus is vaccinia virus.

10. The method of claim 8, wherein said bacterium is attenuated *Vibrio cholerae.*

11. The method of any one of claims 1 to 7, wherein said cell is a mammalian cell.

12. The method of any one of claims 1 to 11, wherein said animal is a mouse.

## Patentansprüche

1. Verfahren zur Herstellung eines gewünschten Polypeptids, RNA-Moleküls oder einer Verbindung, wobei das Verfahren die folgenden Schritte umfasst:
(a) Injizieren eines Mikroorganismus oder einer Zelle, der/die eine klonierte DNA-Sequenz enthält, die das gewünschte Polypeptid oder RNA-Molekül kodiert oder für die Herstellung der Verbindung kodiert, in ein nicht-humanes Tier, das einen Tumor trägt, wobei:
der Tumor ein fester Tumor ist; und
der Mikroorganismus oder die Zelle in dem Tumor akkumuliert;
und
(b) nach dem Töten des Tieres, das Tumorgewebe aus dem Tier entnehmen; und
(c) Isolieren des gewünschten Polypeptids, RNA-Moleküls oder der Verbindung aus dem Tumorgewebe.

2. Verfahren zur Herstellung eines gewünschten Antikörpers, wobei das Verfahren die folgenden Schritte umfasst:
(a) Injizieren eines Mikroorganismus oder einer Zelle, der/die eine klonierte DNA-Sequenz enthält, die ein Antigen kodiert, in ein nicht-humanes Tier, das einen Tumor trägt, wobei:
der Tumor ein fester Tumor ist; und
(b) nach dem Töten des Tieres, den Antikörper, der gegen das Antigen gerichtet ist, aus dem Serum isolieren, das aus dem Tier gewonnen wird.

3. Verfahren zur gleichzeitigen Herstellung eines gewünschten Polypeptids, RNA-Moleküls oder einer Verbindung und eines Antikörpers, der gegen das Polypeptid, RNA-Molekül oder die Verbindung gerichtet ist, wobei das Verfahren die folgenden Schritte umfasst:
(a) Injizieren eines Mikroorganismus oder einer Zelle, der/die eine klonierte DNA-Sequenz enthält, die das gewünschte Polypeptid oder RNA-Molekül kodiert oder für die Herstellung der Verbindung kodiert, in ein nicht-humanes Tier, das einen Tumor trägt, wobei:
der Mikroorganismus oder die Zelle in Tumoren akkumuliert;
und
der Tumor ein fester Tumor ist; und
(b) nach dem Töten des Tieres, das Tumorgewebe aus dem Tier entnehmen; und
(c) Isolieren des gewünschten Polypeptids, RNA-Moleküls oder der Verbindung aus dem Tumorgewebe; und
(d) Isolieren des Antikörpers, der gegen das Polypeptid, RNA-Molekül oder die Verbindung gerichtet ist, aus dem Serum, das aus dem Tier gewonnen wird.

4. Verfahren nach einem der Ansprüche 1 bis 3, wobei der Mikroorganismus oder die Zelle weiterhin ein DNA-Molekül enthält, das ein detektierbares Protein oder ein Protein kodiert, das fähig ist, ein detektierbares Signal zu induzieren.

5. Verfahren nach Anspruch 4, wobei das Protein, das fähig ist, ein detektierbares Signal zu induzieren, ein Lumineszenz- und/oder Fluoreszenz-Protein ist.

6. Verfahren nach Anspruch 5, wobei das Lumineszenz- oder Fluoreszenz-Protein Luciferase, RFP oder GFP ist.

7. Verfahren nach Anspruch 6, wobei der Mikroorganismus oder die Zelle zusätzlich ein Gen enthält, das ein Substrat für eine Luciferase kodiert.

8. Verfahren nach einem der Ansprüche 1 bis 7, wobei der Mikroorganismus ein Bakterium oder ein Virus ist.

9. Verfahren nach Anspruch 8, wobei das Virus Vaccinia-Virus ist.

10. Verfahren nach Anspruch 8, wobei das Bakterium abgeschwächtes *Vibrio cholerae* ist.

11. Verfahren nach einem der Ansprüche 1 bis 7, wobei die Zelle eine Säugetierzelle ist.

12. Verfahren nach einem der Ansprüche 1 bis 11, wobei das Tier eine Maus ist.

## Revendications

1. Procédé pour la production d'un polypeptide, d'une molécule d'ARN ou d'un composé désiré, ledit procédé comprenant les étapes suivantes :
(a) injection d'un microorganisme ou d'une cellule contenant une séquence d'ADN clonée, qui code pour le peptide ou la molécule d'ARN désiré ou qui code pour la production du composé, dans le corps d'un animal non humain porteur d'une tumeur,
la tumeur étant une tumeur solide ; et
le microorganisme ou la cellule s'accumulant dans la tumeur ; et
(b) après sacrifice de l'animal, prélèvement du tissu tumoral dudit animal ; et
(c) isolement du polypeptide, de la molécule d'ARN ou du composé désiré dudit tissu tumoral.

2. Procédé pour la production d'un anticorps désiré, ledit procédé comprenant les étapes suivantes :
(a) injection d'un microorganisme ou d'une cellule contenant une séquence d'ADN clonée codant pour un antigène dans le corps d'un animal non humain porteur d'une tumeur,
le microorganisme ou la cellule s'accumulant dans les tumeurs et exprimant l'antigène dans les tumeurs ; et
la tumeur étant une tumeur solide ; et
(b) après sacrifice de l'animal, isolement de l'anticorps dirigé contre ledit antigène du sérum obtenu à partir dudit animal.

3. Procédé pour la production simultanée d'un polypeptide, d'une molécule d'ARN ou d'un composé désiré et d'un anticorps dirigé contre ledit polypeptide, ladite molécule d'ARN ou ledit composé, ledit procédé comprenant les étapes suivantes :
(a) injection d'un microorganisme ou d'une cellule contenant une séquence d'ADN clonée, qui code pour le polypeptide ou la molécule d'ARN désiré ou qui code pour la production du composé, dans un animal non humain porteur d'une tumeur,
le microorganisme ou la cellule s'accumulant dans les tumeurs ; et
la tumeur étant une tumeur solide ; et
(b) après sacrifice de l'animal, prélèvement du tissu tumoral dudit animal ;
(c) isolement du polypeptide, de la molécule d'ARN ou du composé désiré dudit tissu tumoral ; et
(d) isolement de l'anticorps dirigé contre ledit polypeptide, ladite molécule d'ARN ou ledit composé du sérum obtenu à partir dudit animal.

4. Procédé selon l'une quelconque des revendications 1 à 3, dans lequel ledit microorganisme ou ladite cellule contient de plus une molécule d'ADN codant pour une protéine détectable ou une protéine capable d'induire un signal détectable.

5. Procédé selon la revendication 4, dans lequel ladite protéine capable d'induire un signal détectable est une protéine luminescente et/ou fluorescente.

6. Procédé selon la revendication 5, dans lequel ladite protéine luminescente ou fluorescente est la luciférase, RFP ou GFP.

7. Procédé selon la revendication 6, dans lequel ledit microorganisme ou ladite cellule contient de plus un gène codant pour un substrat pour une luciférase.

8. Procédé selon l'une quelconque des revendications 1 à 7, dans lequel ledit microorganisme est une bactérie ou un virus.

9. Procédé selon la revendication 8, dans lequel ledit virus est le virus de la vaccine.

10. Procédé selon la revendication 8, dans lequel ladite bactérie est *Vibrio cholerae* atténué.

11. Procédé selon l'une quelconque des revendications 1 à 7, dans lequel ladite cellule est une cellule de mammifère.

12. Procédé selon l'une quelconque des revendications 1 à 11, dans lequel ledit animal est une souris.
